# EUROPEAN PATENT APPLICATION

(11) **EP 4 417 603 A1**
(43) Date of publication of application: **21.08.2024**
(21) Application number: 22880403.5
(22) Date of filing: 14.10.2022
(51) Int. Cl.: C07D 405/14, C07C 63/70, C07C 63/44, A61K 31/4433, A61P 35/00

(54) **METHOD FOR PREPARING BENZOFURAN DERIVATIVE**

(30) Priority: 15.10.2021 CN 202111206101
(71) Applicant: Jiangsu Hengrui Pharmaceuticals Co., Ltd., Lianyungang, Jiangsu 222047 (CN)
(72) Inventor: CHEN, Ya, Lianyungang, Jiangsu 222047 (CN); WANG, Ruzhi, Lianyungang, Jiangsu 222047 (CN); WANG, Yang, Lianyungang, Jiangsu 222047 (CN); XIE, Ting, Lianyungang, Jiangsu 222047 (CN); ZHANG, Lei, Lianyungang, Jiangsu 222047 (CN); GUO, Changshan, Lianyungang, Jiangsu 222047 (CN)
(74) Representative: D Young & Co LLP
(86) International application number: PCT/CN2022/125302
(87) International publication number: WO 2023/061467

(57) **Abstract**

A method of preparing a benzofuran derivative. Specifically, this relates to a preparation method of a benzofuran derivative as shown in Formula I, which greatly improves yield and has good application prospects.

## Description

This application claims priority rights for Chinese patent application 2021112061010 with an application date of October 15, 2021. This application cites the full text of the aforementioned Chinese patent application.

### Technical Field

The present disclosure relates to a method of preparing benzofuran derivatives, which belongs to the field of pharmaceutics.

### Background Art

Lymphoma is a malignant tumor originating from the lymphohematopoietic system. Based on its tumor cells, it is divided into two types: non-Hodgkin's lymphoma (NHL) and Hodgkin's lymphoma (HL). In Asia, 90% of patients are NHL, and pathologically, it mainly involves the different degrees of differentiation of lymphocytes, histiocytes or reticulocytes. Based on the natural course of NHL, it can be classified into three clinical types, namely highly aggressive, aggressive, and indolent lymphoma. Based on different lymphocyte origins, it can be divided into B-cell, T-cell, and NK (natural killer) cell lymphoma. The main function of B cells is to secrete various antibodies to help the body defend against various exogenous invasions.

The histone methyltransferase encoded by the EZH2 gene is a catalytic component of the polycombin inhibitory complex 2 (PRC2). EZH2 levels are abnormally elevated in cancerous tissues compared to normal tissues, while EZH2 expression levels are highest in advanced cancer or poor prognosis. In some cancer types, EZH2 overexpression occurs concurrently with amplification of the EZH2 gene. Numerous si/shRNA experimental studies have found that reducing EZH2 expression in tumor cell lines inhibits tumor cell proliferation, migration, and invasion or angiogenesis, and leads to apoptosis.

An EZH2 inhibitor is provided in WO2017084494A with the structure shown below:

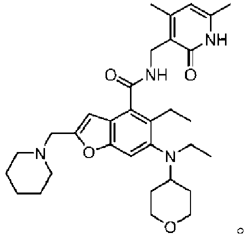

WO2019091450A also discloses a method of preparing a previously described compound, and the present disclosure provides a new method of preparing a pharmaceutically acceptable salt of the compound in consideration of simplifying the preparation process and reducing production costs.

### Summary of the Invention

The present disclosure provides a method of preparing a compound of Formula V or a pharmaceutically acceptable salt thereof, comprising the step of reacting the compound of Formula VI with the compound of Formula VII: 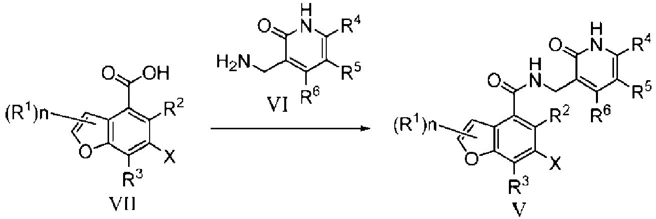 wherein:
R¹ is the same or different, each independently selected from halogen, alkyl, alkoxy, amino, nitro, hydroxy, cyano, cycloalkyl, heterocycloalkyl, aryl, and heteroaryl; the alkyl, alkoxy, cycloalkyl, or
heterocycloalkyl optionally substituted with one or more R^{A}; the R^{A} is selected from halogen, hydroxy, cyano, amino, nitro, alkyl, alkoxy, cycloalkyl, and heterocycloalkyl;
R², R³, R⁴, R⁵, R⁶ are each independently selected from hydrogen, halogen, alkyl, alkoxy, amino, nitro, hydroxy, cyano, cycloalkyl, and heterocycloalkyl; the alkyl, alkoxy, cycloalkyl, heterocycloalkyl is optionally substituted with one or more R^{B}; the R^{B} is selected from halogen, hydroxy, cyano, amino, and nitro;
n is selected from 1 or 2;
X is selected from halogen.

In an optional embodiment, the method of preparing a compound of formula V or a pharmaceutically acceptable salt thereof, wherein the compound of formula VI reacts with the compound of formula VII under the action of a condensing agent.

In an optional embodiment, the condensing agent is selected from N,N-carbonyldiimidazole (CDI), dicyclohexylcarbodiimide (DCC), diisopropylcarbodiimide (DIC), 1-(3-dimethylaminopropyl)-3-ethylcarbodiimide (EDCI), 4-dimethylaminopyridine (DMAP), 4-pyrrolidinylpyridine (4-PPY), 1-hydroxybenzotriazole (HOBT, 1-hydroxy-7-azabenzotriazole (HOAT), 2-(7-azabenzotriazole)-N,N',N',N'-tetramethylureahexafluorophosphate (HATU), and O-benzotriazole-tetramethylureahexafluorophosphate (HBTU).

In an optional embodiment, the condensing agent is selected from a combination of EDCI and HOBT.

In an optional embodiment, the compound of Formula V or a pharmaceutically acceptable salt thereof is prepared and reacted in a basic environment.

In an optional embodiment, the method of preparing a compound of Formula V or a pharmaceutically acceptable salt thereof provides a basic environment selected from the group consisting of triethylamine, pyridine, and N,N-diisopropylethylamine (DIPEA).

In an optional embodiment, in the method of preparing a compound of formula V in the present disclosure or a pharmaceutically acceptable salt thereof, the reaction solvent is selected from N,N-dimethylformamide (DMF) or dichloromethane.

In an optional embodiment, the method of preparing a compound of formula V or a pharmaceutically acceptable salt thereof, comprising the step of reacting Compound 1 with 3-(aminomethyl)-4,6-lutidine-2(1H)-one to obtain Compound 2:

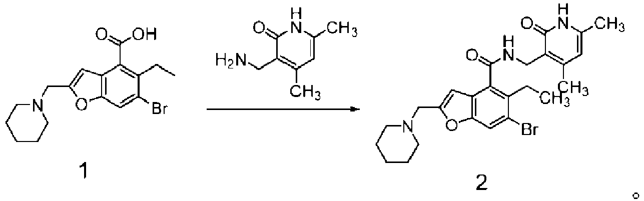

Another aspect of the present disclosure provides a method of preparing a compound of Formula III or a pharmaceutically acceptable salt thereof, which comprises the step of reacting the compound of Formula V with the compound of Formula IV: 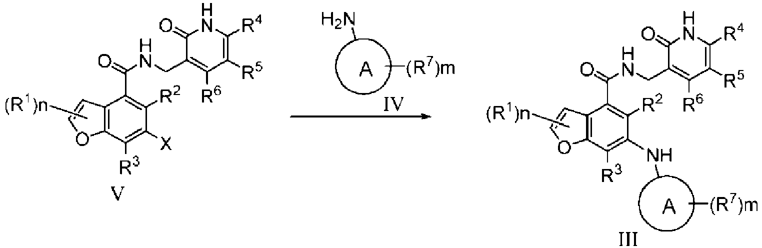 wherein:
ring A is selected from cycloalkyl and heterocycloalkyl;
R⁷ is selected from halogen, alkyl, alkoxy, amino, nitro, hydroxy, cyano, cycloalkyl, heterocycloalkyl, aryl, and heteroaryl; the alkyl, alkoxy, cycloalkyl, heterocycloalkyl, aryl, or heteroaryl is optionally substituted with one or more R^{c}; the R^{c} is selected from halogen, alkyl, alkoxy, amino, nitro, hydroxy, cyano, cycloalkyl, heterocycloalkyl, aryl, and heteroaryl;
m is selected from 0, 1, 2, 3, 4, 5, or 6;
R¹, R², R³, R⁴, R⁵, R⁶, and n are defined in the compounds of formula V, respectively.

In an optional embodiment, the compound of Formula V in the preparation method of a compound of Formula III or a pharmaceutically acceptable salt thereof reacts with the compound of Formula IV in the presence of a palladium catalyst and a phosphine ligand catalyst.

The palladium catalyst in an optional embodiment is bis(dibenzylideneacetone)palladium (Pd(dba)₂).

In an optional embodiment, the phosphine ligand catalyst is R-(+)-2,2'-bis(diphenylphosphine)-1,1'-binaphthalene (BINAP).

In an optional embodiment, a compound of Formula III or a pharmaceutically acceptable salt thereof is reacted in a basic environment.

In an optional embodiment, the method of preparing a compound of Formula III or a pharmaceutically acceptable salt thereof provides a basic environment selected from potassium tert-butanol (tBuOK) and/or sodium tert-butanol (tBuONa).

In an optional embodiment, the method of preparing a compound of formula III or a pharmaceutically acceptable salt thereof comprises the step of reacting Compound 2 with 4-aminotetrahydropyran to obtain Compound 3:

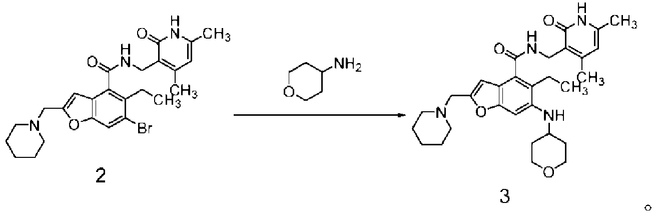

In an optional embodiment, the method of preparing a compound of Formula III provided in the present disclosure or a pharmaceutically acceptable salt thereof, further comprising the steps in the method of preparing the aforementioned compound of Formula V or a pharmaceutically acceptable salt thereof.

Another aspect of the present disclosure provides a method of preparing a compound of Formula I or a pharmaceutically acceptable salt thereof, which comprises the step of reacting the compound of Formula III with the compound of Formula II: 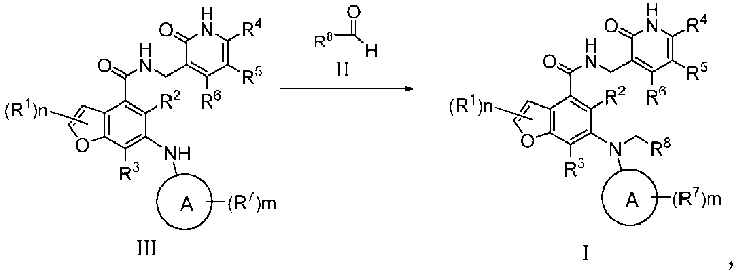 wherein:
R⁸ is alkyl;
R¹, R², R³, R⁴, R⁵, R⁶, R⁷, Ring A, n, and m are defined in the compounds of Formula III, respectively.

In an optional embodiment, the method of preparing the compound of Formula I or a pharmaceutically acceptable salt thereof is reacted under a weak acid environment.

In an optional embodiment, in the method of preparing a compound of Formula I or a pharmaceutically acceptable salt thereof, the weak acidic environment is provided by acetic acid. In an optional embodiment, in the method of preparing a compound of Formula I or a pharmaceutically acceptable salt thereof, the compound of Formula III reacts with a compound of Formula II in the presence of a reducing agent.

In an optional embodiment, the reducing agent is selected from sodium borohydride or sodium cyanoborohydride.

In an optional embodiment, the method of preparing a compound of Formula I or a pharmaceutically acceptable salt thereof, comprising the step of reacting Compound 3 with acetaldehyde to obtain Compound 4:

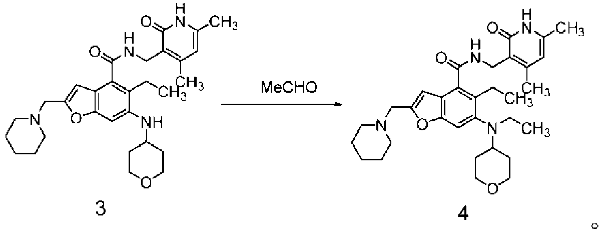

In an optional embodiment, the method of preparing a compound of Formula I provided in the present disclosure or a pharmaceutically acceptable salt thereof, further comprising the steps in the aforementioned method of preparing a compound of Formula V or a pharmaceutically acceptable salt thereof and/or the steps in the aforementioned method of preparing a compound of Formula III or a pharmaceutically acceptable salt thereof.

In an optional embodiment, the present disclosure provides a method of preparing a compound of Formula I or a pharmaceutically acceptable salt thereof, which comprises the steps of: 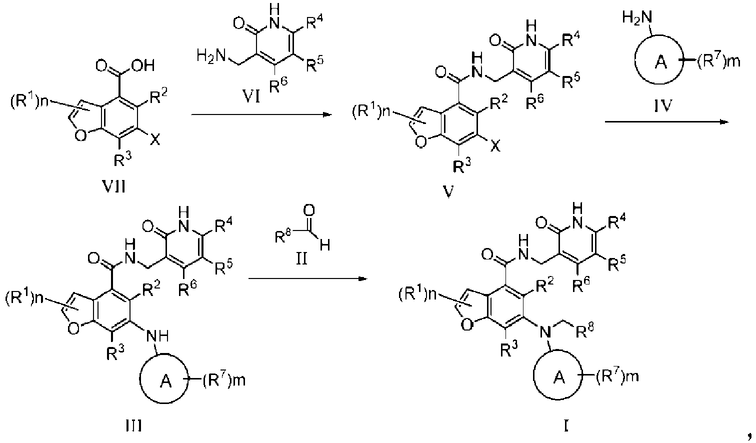 wherein R¹, R², R³, R⁴, R⁵, R⁶, R⁷, ring A, n, and m are as previously described, respectively. In an optional embodiment, the method of preparing a compound of Formula I provided in the present disclosure or a pharmaceutically acceptable salt thereof, comprising the steps of:

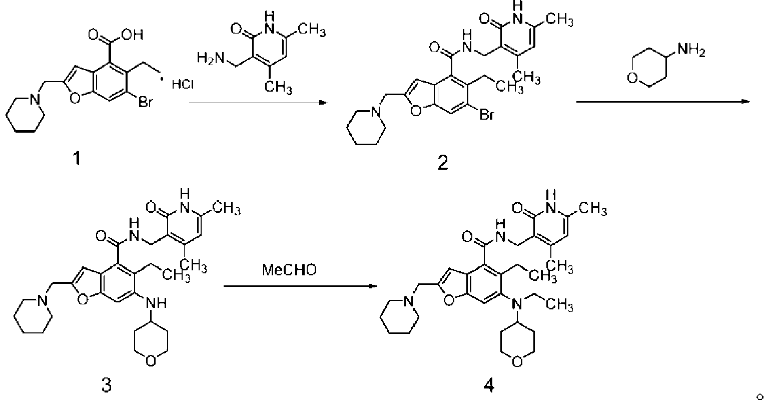

According to another aspect of the present disclosure, a compound of Formula III or a pharmaceutically acceptable salt thereof is provided: 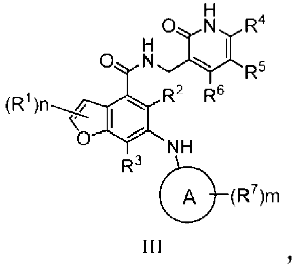 wherein R¹, R², R³, R⁴, R⁵, R⁶, R⁷, ring A, n, and m are as previously described, respectively. In an optional embodiment, the compound of Formula III or a pharmaceutically acceptable salt thereof provided in the present disclosure are:

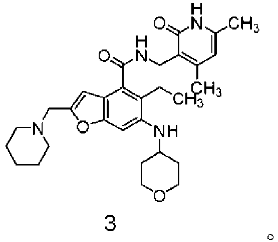

According to another aspect of the present disclosure, a compound of Formula V or a pharmaceutically acceptable salt thereof is provided: 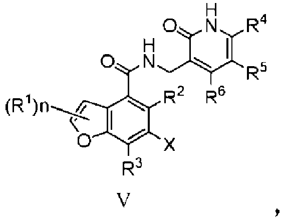 wherein R¹, R², R³, R⁴, R⁵, R⁶, n, and m are as previously described, respectively.

In an optional embodiment, a compound of Formula V provided in the present disclosure or a pharmaceutically acceptable salt thereof, which is:

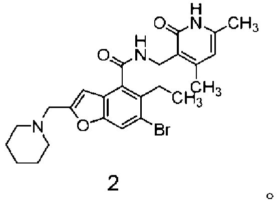

### Detailed Description of the Invention

Unless stated to the contrary, terms used in the Specification and Claims have the following meanings.

The term "alkyl" refers to a saturated aliphatic hydrocarbon group, which is a linear or branched group comprising 1 to 20 carbon atoms, preferably an alkyl group comprising 1 to 12 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, n-hexyl, n-octyl, n-heptyl, isooctyl, decyl, undecyl, dodecyl, and various branched isomers thereof, and the like.

The term "cycloalkyl" refers to a saturated or partially unsaturated monocyclic or polycyclic hydrocarbon substituent, with the cycloalkyl ring comprising 3 to 20 carbon atoms, preferably 3 to 12 carbon atoms, preferably 3 to 10 carbon atoms, more preferably 3 to 6 carbon atoms. Non-limiting examples of monocycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycycloalkyl groups include cycloalkyl groups of spirocyclic, fused, and bridged rings.

The term "heterocycloalkyl" refers to a saturated or partially unsaturated monocyclic or polycyclic hydrocarbon substituent comprising 3 to 20 ring atoms, wherein one or more ring atoms are heteroatoms selected from nitrogen, oxygen, or S(O)ₘ (wherein m is an integer 0 to 2), but excludes a ring portion of -O-O-, -O-S-, or -S-S-, while the remaining ring atoms are carbon. Preferably, it comprises 3 to 12 ring atoms, wherein 1 to 4 are heteroatoms. More preferably, it comprises 3 to 10 ring atoms, wherein 1-4 are heteroatoms. More preferably, it comprises 5 to 6 ring atoms, wherein 1-3 are heteroatoms. Non-limiting examples of monocyclic heterocyclyl groups include pyrrolidinyl, tetrahydropyranyl, 1,2,3,6-tetrahydropyridyl, piperidinyl, piperazinyl, morpholinyl, thiomorpholinyl, homopiperazinyl, and the like. Polycyclic heterocyclyl groups include heterocyclyl groups of a spirocyclic ring, a fused ring, and a bridge ring.

The heterocyclyl ring may be fused to an aryl, heteroaryl, or cycloalkyl ring, wherein the ring connected with the parent structure is a heterocyclyl group, non-limiting examples of which include:

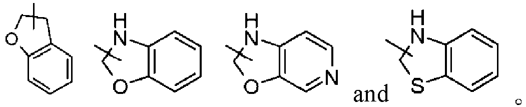

The term "aryl" refers to a 6- to 14-membered all-carbon monocyclic or fused polycyclic group having a conjugated π electron system (that is, a ring sharing adjacent carbon atom pairs), preferably 6- to 10-membered, such as phenyl and naphthyl. The aryl ring may be fused to a heteroaryl, heterocyclyl, or cycloalkyl ring, wherein the ring connected with the parent structure is an aryl ring, non-limiting examples of which include: 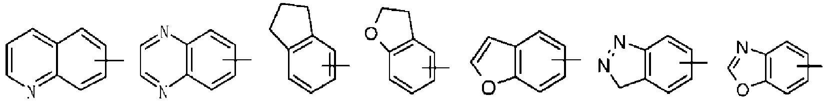 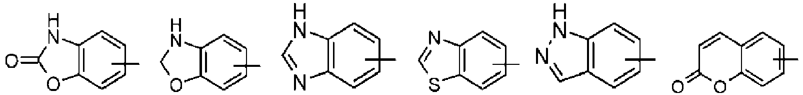 and

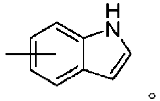

The aryl group may be substituted or unsubstituted, and when substituted, the substituent is preferably one or more of the following groups independently selected from one or more substituents of halogen, alkyl, alkoxy, haloalkyl, hydroxyl, hydroxyalkyl, cyano, amino, nitro, cycloalkyl, and heterocyclyl.

The term "heteroaryl" refers to a heteroaromatic system comprising 1 to 4 heteroatoms, 5 to 14 ring atoms, wherein the heteroatoms are selected from oxygen, sulfur, and nitrogen. Heteroaryl groups are preferably those with 5 to 10 members, such as furanyl, thienyl, pyridyl, pyrrolyl, N-alkylpyrrolyl, pyrimidinyl, pyrazinyl, pyridazinyl, imidazolyl, pyrazolyl, tetrazolyl, and the like. The heteroaryl ring may be fused to an aryl, heterocyclyl, or cycloalkyl ring, wherein the ring connected with the parent structure is a heteroaryl ring, non-limiting examples of which include:

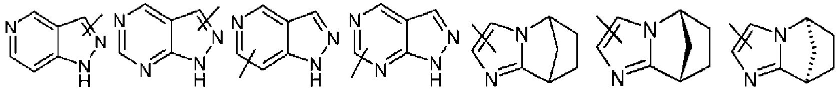

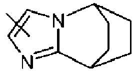

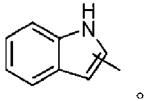

The term "alkoxy" refers to -O-(alkyl) and -O-(unsubstituted cycloalkyl), wherein the definition of alkyl is as described above. Non-limiting examples of alkoxy groups include: Methoxy, ethoxy, propoxy, butoxy, cyclopropoxy, cyclobutoxy, cyclopentoxy, and cyclohexoxy. The alkoxy group may be optionally substituted or unsubstituted, and when substituted, the substituent is preferably one or more of the following groups independently selected from one or more substituents of halogen, alkyl, alkoxy, haloalkyl, hydroxyl, hydroxyalkyl, cyano, amino, nitro, cycloalkyl, heterocyclyl, aryl, and heteroaryl.

The term "haloalkyl" refers to an alkyl substituted with one or more halogens, wherein the alkyl is as defined above. The term "hydroxyl" refers to a -OH group.

The term "hydroxyalkyl" refers to an alkyl substituted with a hydroxyl group, wherein the alkyl group is as defined above.

The term "halogen" refers to fluorine, chlorine, bromine, or iodine.

The term "amino" refers to -NH₂.

The term "cyano" refers to -CN.

The term "nitro" refers to -NO₂.

The term "oxo" refers to =O.

In the chemical structure of the compound described in the present disclosure, the bond "j" represents an unspecified configuration, i.e., if a chiral isomer is present in the chemical structure, the bond "/" may be " 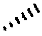 " or " 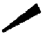 ", or both " 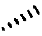 " and " 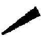 " configurations. In the chemical structure of the compounds described in the present disclosure, the bond "∥" does not specify a configuration, i.e., can be in the Z configuration or the E configuration or both configurations. Compounds and intermediates of the disclosure may also be present in different tautomer forms, and all such forms are included within the scope of the disclosure. The term "tautomer" or "tautomer form" refers to structural isomers of different energies that can be tautomerized via a low energy barrier. For example, proton tautomers (also referred to as proton transfer tautomers) include tautomerization via proton migration, such as keto-enol, imine-enamine, and lactam-lactimide isomerization. An example of lactam-lactimide equilibrium is shown between A and B below:

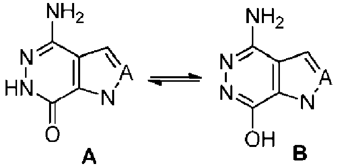

All compounds in the present disclosure may be drawn as Type A or Type B. All tautomeric forms are within the scope of the disclosure. No tautomers are excluded from compound naming.

The pharmaceutically acceptable salts of the present disclosure include but are not limited to solvates, and the solvents include but are not limited to water, methanol, ethanol, isopropanol, acetonitrile, acetone, tetrahydrofuran, ethyl acetate, n-propanol, 2-butanone, propylene glycol monomethyl ether, n-heptane, cyclohexane, and n-hexane.

"Optional" or "optionally" means that the subsequently described event or environment may but need not occur, with the description including the event or environment occurring or not occurring in a location setting. For example, "optionally substituted with an alkyl group" means that an alkyl group may but does not have to be present, including situations where the heterocyclic group is substituted with an alkyl group and situations where the heterocyclic group is not substituted with an alkyl group.

"Substituted" refers to one or more hydrogen atoms in a group, preferably up to 5, more preferably 1 to 3 hydrogen atoms independently of each other, being substituted with a respective number of substituents. It goes without saying that substituents are only in their possible chemical locations, and those skilled in the art can determine (by experiment or theory) possible or unlikely substitutions without much effort. For example, an amino group or hydroxyl group having free hydrogen may be unstable when bound to a carbon atom having an unsaturated (e.g., ethylenically) bond.

A "pharmaceutical composition" refers to a mixture of one or more compounds described herein or physiologically/pharmaceutically acceptable salts or prodrugs thereof with other chemical components, as well as other components such as physiologically/pharmaceutically acceptable carriers and excipients. The purpose of the pharmaceutical composition is to promote the administration to the organism to facilitate the absorption of the active ingredient and thereby exert biological activity. The purity or content described in the present disclosure is determined by HPLC detection, and the compound characterization data is obtained by the analysis of the nuclear magnetic resonance spectrum. The reagents used in the present disclosure can be purchased through commercial channels.

### Specific Embodiment(s)

The present disclosure will be explained in more detail below with reference to embodiments, which are only used to illustrate the technical solution of the present disclosure, and the substance and scope of the present disclosure are not limited thereto.

The compound structure of the present disclosure is determined by nuclear magnetic resonance (NMR) or/and mass spectrometry (MS). NMR displacement (shift R is given in units of 10-6 (ppm). The determination of NMR was performed using a Bruker AVANCE-400 nuclear magnetic resonance instrument. The determination solvents were deuterated dimethyl sulfoxide (DMSO-d₆), deuterated chloroform (CDCl₃), and deuterated methanol (CD₃OD), and the internal standard was tetramethylsilane (TMS).

FINNIGANLCQAd (ESI) mass spectrometer (manufacturer: Thermo, model: Finnigan LCQ advantage MAX) was used for the determination of MS.

HPLC was determined using the WATER e2695-2489 high performance liquid chromatograph. Known starting materials of the present disclosure may be synthesized using or in accordance with methods known in the art, or may be purchased from companies such as BEPHARM.

### Preparation of Compound 4 of Embodiment 1

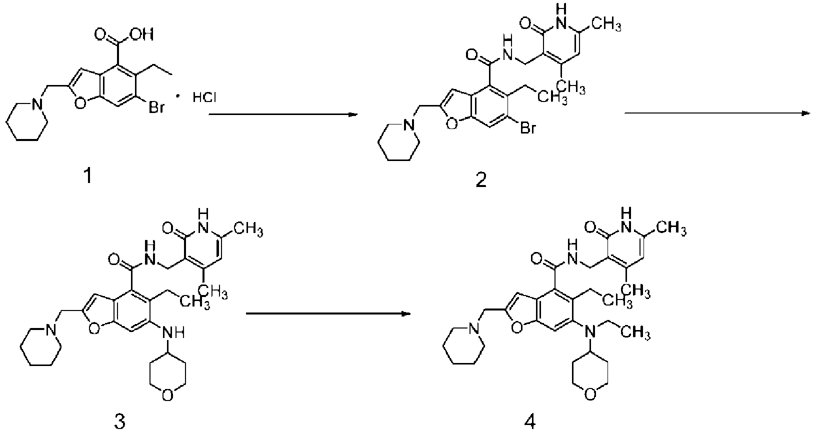

### Step 1. Synthesis of Compound 2

4 L of DMF, 500 g of the compound of Formula 1, and 800 g of DIPEA were added sequentially into the reaction vessel. After stirring, 335.5 g of HOBt, 476 g of EDCI, and 257.5 g of 3-(aminomethyl)-4,6-lutidine-2(1H)-one were added sequentially, and then 1 L of DMF was added. It was heated and warmed to an internal temperature of 40 °C, and it was stirred until completely reacted. Water was added to precipitate solids, and it was shake filtered after beating. It was washed with water and then dried to obtain the compound of Formula 2 (580 g), with a yield of 94%.
¹H NMR (400 MHz, DMSO-*d*₆) δ 11.20 (s, 1H), 8.74 (t, *J* = 4.8, 1H), 7.99 (d, *J* = 0.4, 1H), 7.08 (s, 1H), 6.26 (s, 1H), 5.40-4.52 (m, 2H), 4.43-4.42 (m, 2H), 3.41 - 3.38 (m, 2H), 2.97-2.92 (m, 2H), 2.81-2.76 (m, 2H), 2.54 (t, *J* = 2.0, 3H), 2.35 (s, 3H), 1.87-1.80 (m, 4H), 1.69-1.65 (m, 1H), 1.39-1.28 (m, 1H), 1.10 (t, J =7.6, 3H) ppm _{∘}
LCMS (m/z): 500.36 [M+H]⁺_{∘}

### Step 2. Synthesis of Compound 3

0.46 g of Pd(dba)₂ and 2.0 g of BINAP were weighed out. It was mixed well and added into the reaction vessel. Then 20.0 g of the compound of Formula 2, 11.52 g of *^{t}* BuONa, and 3.2 g of *^{f}* BuOLi were added, with vacuum -N₂ replacement. 200 mL of 1,4-dioxane under N₂ protection was added, and then 8.08 g of 4-aminotetrahydropyran was added. It was heated up to 100 °C while stirring to reflux the reaction for 24 hours until the reaction was complete, and then the posttreatment was started.

Water was added to quench the reaction solution, and the aqueous phase was washed with DCM. The organic phase was discarded, and the aqueous phase was retained. Subsequently, DCM was added to the aqueous phase for extraction, the aqueous phase was discarded, and the organic phase was retained. NaHSO₃ aqueous solution was added, and the internal temperature was increased to 30-40 °C. It was stirred for 1 hour and the separation solution was allowed to stand. The organic phase was separated. It was dried, vacuum filtered, and spun dry to obtain the crude compound of Formula 3.

MTBE was added, and it was stirred, refluxed, and beat. Afterwards, n-heptane was added, and it was cooled and crystallized. The mother liquor was shake filtered, and the filter cake was rinsed with n-heptane. After drying, the compound of Formula 3 (17.3 g) was obtained, and the yield was 83%.
¹H NMR (400 MHz, CDCl₃) δ 12.80 (s, 1H), 7.13 (t, *J* = 5.6, 111), 6.77 (s, 1H), 6.36 (s, 1H), 5.93 (s, 1H), 4.59 (d, *J* = 6.0, 2H), 4.03-3.98 (m, 2H), 3.64-3.62 (m, 2H), 3.57-3.51 (m, 3H), 3.45 (s, 2H), 2.62 (dd, *J* = 7.6, 2H), 2.4 (s, 3H), 2.35 (s, 3H), 2.09-2.06 (m, 5H), 1.54-1.46 (m, 6H), 1.19-1.15 (m, 3H), 1.35-1.26 (m, 2H), 1.19-1.15 (m, 3H) ppm_{∘}
LCMS (m/z): 521.05 [M+H]⁺_{∘}

### Step 3.Synthesis of Compound 4

50 mL of DCM was added to the reaction vessel, and 5.0 g of the compound of Formula 3 was added while stirring. It was dissolved and reduced to 0-10 °C. Acetaldehyde 2.11 g and acetic acid 0.576 g were added successively, and it was stirred for 0.5 hours. Then 6.31 kg of sodium borohydride acetate was added, slowly increasing to 25 °C under N₂ protection until the reaction was complete. Water, NaOH, liquid separation, and saturated sodium bicarbonate were added for washing, drying, filtering, and spin drying. Methyl tert-butyl ether was added to the vessel, and it was stirred and dissolved and then heated and refluxed. n-heptane was dripped in, and it was cooled and crystallized and filtered to obtain the crude compound of Formula 4 (5.0 g), with a yield of 95%.
¹H NMR (400 MHz, CDCh) δ 12.98 (s, 1H), 7.28-7.27 (m, 1H), 7.10 (t, *J* =5.2, 1H), 6.50 (s, 1H), 5.94 (s, 1H), 4.61 (d, *J* =5.6, 2H), 3.94 (d, *J* =10.8, 2H), 3.51 (s, 2H), 3.28 (t, *J* =10.4, 2H), 3.05 (dd, *J* =6.8, 2H), 2.97-2.87 (m, 3H), 2.43-2.39 (m, 7H), 2.17-2.10 (m, 3H), 1.69-1.64 (m, 4H), 1.55 (s, 4H), 1.37 (s, 2H), 1.05 (t, *J* =7.2, 3H), 0.87 (t, *J* =6.8, 3H) ppm_{∘}
LCMS (m/z): 549.25 [M+H]⁺_{∘}

## Claims

1. A method of preparing a compound of Formula V or a pharmaceutically acceptable salt thereof, comprising the step of reacting the compound of Formula VI with the compound of Formula VII: 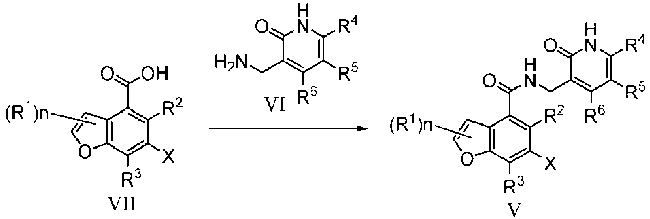 wherein:
R¹ is the same or different, each independently selected from halogen, alkyl, alkoxy, amino, nitro, hydroxy, cyano, cycloalkyl, heterocycloalkyl, aryl, and heteroaryl, the alkyl, alkoxy, cycloalkyl, or
heterocycloalkyl optionally substituted with one or more R^{A}; the R^{A} is selected from halogen, hydroxy, cyano, amino, nitro, alkyl, alkoxy, cycloalkyl, and heterocycloalkyl;
R², R³, R⁴, R⁵, R⁶ are each independently selected from hydrogen, halogen, alkyl, alkoxy, amino, nitro, hydroxy, cyano, cycloalkyl, and heterocycloalkyl; the alkyl, alkoxy, cycloalkyl,
heterocycloalkyl is optionally substituted with one or more R^{B}; the R^{B} is selected from halogen, hydroxy, cyano, amino, and nitro;
n is selected from 1 or 2;
X is selected from halogen.

2. The method of preparing a compound of formula V or a pharmaceutically acceptable salt thereof according to Claim 1, comprising the step of reacting Compound 1 with 3-(aminomethyl)-4,6-lutidine-2(1H)-one to obtain Compound 2:

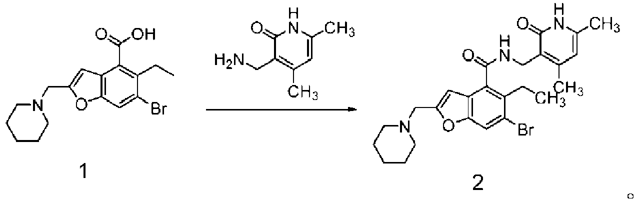

3. A method of preparing a compound of Formula III or a pharmaceutically acceptable salt thereof, comprising the step of reacting a compound of Formula V with a compound of Formula IV, 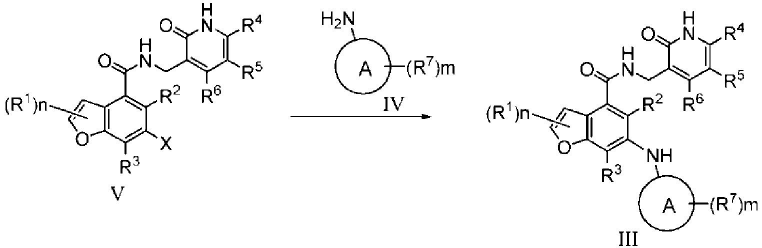 wherein:
ring A is selected from cycloalkyl and heterocycloalkyl;
R⁷ is selected from halogen, alkyl, alkoxy, amino, nitro, hydroxy, cyano, cycloalkyl, heterocycloalkyl, aryl, and heteroaryl, the alkyl, alkoxy, cycloalkyl, heterocycloalkyl, aryl, or
heteroaryl is optionally substituted with one or more R^{c}, the R^{c} is selected from halogen, alkyl, alkoxy, amino, nitro, hydroxy, cyano, cycloalkyl, heterocycloalkyl, aryl, and heteroaryl;
m is selected from 0, 1, 2, 3, 4, 5, or 6;
R¹, R², R³, R⁴, R⁵, R⁶, and n are as set forth in Claim 1, respectively.

4. The method of preparing a compound of Formula III or a pharmaceutically acceptable salt thereof according to Claim 3, comprising the step of reacting Compound 2 with 4-aminotetrahydropyran to obtain Compound 3:

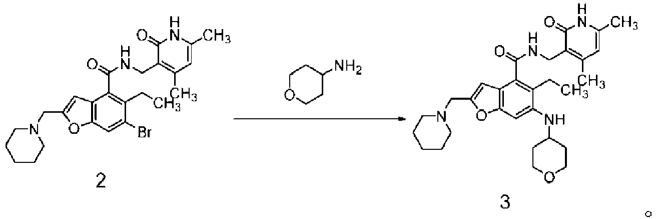

5. The method of preparing a compound of Formula III or a pharmaceutically acceptable salt thereof according to any one of Claims 3-4, further comprising the step in the method of preparing a compound of Formula V or a pharmaceutically acceptable salt thereof of any one of Claims 1-2.

6. A method of preparing a compound of Formula I or a pharmaceutically acceptable salt thereof, comprising the step of reacting the compound of Formula III with the compound of Formula II: 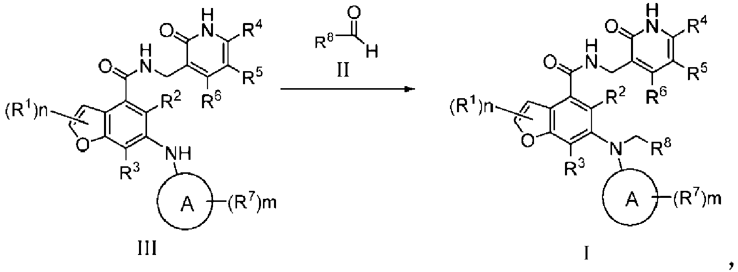 wherein:
R⁸ is alkyl;
R¹, R², R³, R⁴, R⁵, R⁶, R⁷, Ring A, n, and m are as set forth in Claim 3, respectively.

7. The method of preparing a compound of formula I or a pharmaceutically acceptable salt thereof according to Claim 6, comprising the step of reacting Compound 3 with acetaldehyde to obtain Compound 4:

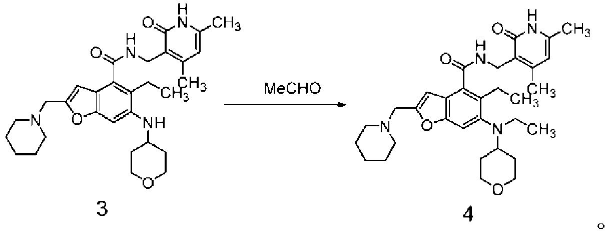

8. The method of preparing the compound of Formula I or a pharmaceutically acceptable salt thereof according to any of Claims 6-7, further comprising the steps in the method of preparing the compound of Formula V or a pharmaceutically acceptable salt thereof of any of Claims 1-2 and/or the steps in the method of preparing the compound of Formula III or a pharmaceutically acceptable salt thereof of any of Claims 3-4.

9. The method of preparing the compound of Formula I or a pharmaceutically acceptable salt thereof according to Claim 8, comprising the step of:

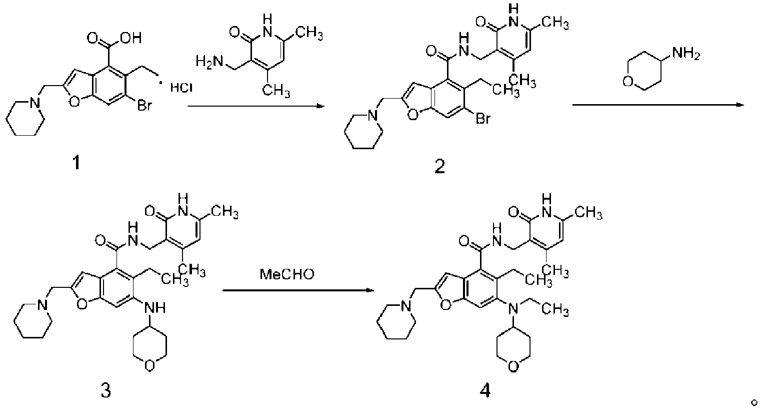

10. A compound of Formula III or a pharmaceutically acceptable salt thereof: 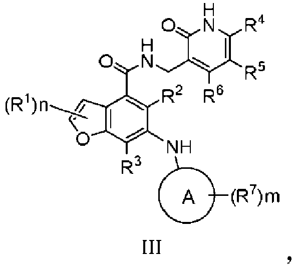 wherein, R¹, R², R³, R⁴, R⁵, R⁶, R⁷, ring A, n, and m are as set forth in Claim 3, respectively.

11. The compound of Formula III or a pharmaceutically acceptable salt thereof according to Claim 10, which is:

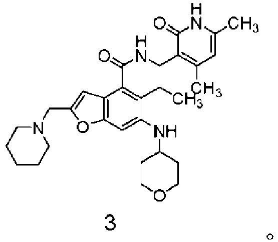

12. A compound of Formula V or a pharmaceutically acceptable salt thereof: 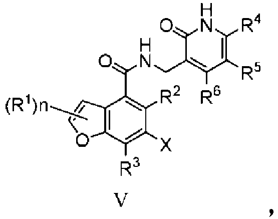 wherein, R¹, R², R³, R⁴, R⁵, R⁶, n, and m are as described in Claim 1, respectively.

13. The compound of formula V or a pharmaceutically acceptable salt thereof according to Claim 12, which is:

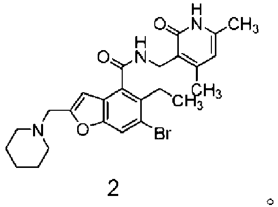

14. A pharmaceutical composition, with the pharmaceutical composition containing a therapeutically effective amount of a compound or a pharmaceutically acceptable salt thereof prepared by any of the methods of Claims 6-9 to obtain the compound of Formula I and one or more pharmaceutically acceptable carriers, diluents, or excipients.
